# EUROPEAN PATENT SPECIFICATION

(11) **EP 4 418 985 B1**
(45) Date of publication and mention of the grant of the patent: **26.11.2025**
(21) Application number: 21798669.4
(22) Date of filing: 22.10.2021
(51) Int. Cl.: A61B 5/00, A61B 5/0215, A61F 2/88

(54) **IMPLANT FOR PRESSURE SENSING INSIDE A BODY LUMEN**
IMPLANTAT FÜR DRUCKMESSUNG IN EINEM KÖRPERLUMEN
IMPLANT POUR LA DÉTECTION DE PRESSION À L'INTÉRIEUR D'UN CONDUIT CORPOREL

(43) Date of publication of application: 28.08.2024
(73) Proprietor: Angiomed GmbH & Co. Medizintechnik KG, 76227 Karlsruhe (DE)
(72) Inventor: HALDER, Sudeepa, 76227 Karlsruhe (DE); VASILJEVIC, Mladen, 76227 Karlsruhe (DE); WONG, Kai Chun, 76227 Karlsruhe (DE); VOGEL, Michael, 76227 Karlsruhe (DE); DIETRICH, Daniel, 76227 Karlsruhe (DE); BELKOUCH, Amal, 76227 Karlsruhe (DE)
(74) Representative: Hoffmann Eitle
(86) International application number: PCT/EP2021/079358
(87) International publication number: WO 2023/066501

(56) References cited:
- WO-A1-2014/159991
- US-A1- 2008 058 652
- US-A1- 2019 269 339
- US-A1- 2019 343 388

## Description

### Technical Field

The present invention relates to an implant for determining a pressure difference inside a bodily lumen.

### Technical Background

In a number of medical applications, it is advantageous to measure a pressure difference between different points inside a patient's body. For example, if a stent/stent graft is implanted in a patient's vasculature, it would be advantageous to be able to measure the pressure difference between the two longitudinal ends of the implant. This applies in particular when using stent grafts for a transjugular intrahepatic portosystemic shunt (TIPS), where the monitoring of the portal pressure gradient (PPG) in patients with portal hypertension managed with TIPS is of clinical relevance.

For a conventional TIPS procedure, the gold standard to measure the portal pressure gradient is the hepatic venous pressure gradient (HVPG) measurement technique prior to TIPS implantation or a venography that is performed post-TIPS implantation. That technique cannot, however, be carried out permanently since it would require a continuous intervention on the patient. During that TIPS procedure, pressure gradients can be measured to help the physicians select the optimal shunt diameter. However, the pressure measurements require the assistance of a pressure catheter or of in vivo pressure sensors. Such measurement methods are generally rather invasive and should thus, if possible, be avoided.

Further, conventional TIPS requires follow-up visits with ultrasound examinations every 3 months in the first year after implantation and every 6 months thereafter. However, such ultrasound examinations are difficult to perform in TIPS patients. Since, typically, at least 25% of the patients suffer a shunt dysfunction within the first two years after TIPS placement, the number of follow-up visits and the TIPS monitoring frequency should be increased. It is also the case that currently, about 10% of the patients have a new bleeding episode, and approximately 30% have varying degrees of hepatic encephalopathy in which an inappropriate portal pressure gradient could be one of the causes. It would thus be highly desirable to have a different way of monitoring the performance of a TIPS shunt (or, in fact, of any stent/stent graft) post-implantation. It would also be desirable to be able to make those follow-up visits faster and less burdensome for patients.

Whilst we have described above issues that generally only arise with TIPS shunts, it is also the case that the monitoring of other blood vessels and, in fact, bodily lumens in general (e.g. bowels, urethra, ...) could be useful for a surgeon who wants to monitor the performance of implants deployed in them or who is interested in monitoring the pressure drops that occur in them rather generally.

In particular, it would be advantageous if such a pressure difference could be measured without having to perform an invasive surgery on the patient. It would, of course, be possible to introduce, if the blood pressure difference between two points is to be measured, a central venous catheter into the patient's vasculature and to measure the pressure difference between those two points. However, the corresponding procedure is highly invasive and is thus complicated to perform. This therefore restricts the use of such procedures to only those situations where it is clinically urgently required to perform such a measurement. However, it would also be beneficial to be able to measure such a pressure difference less invasively, since this would enable a surgeon to notice any unusual developments in the pressure difference much earlier, so that the pressure difference could be used as an early warning system for a deterioration in a patient's health.

US 2019/269339 A1 discloses features falling under the preamble of claim 1. WO 2014/159991 A1 and US 2008/058652 A1 are further prior art.

### Summary of the Invention

The present invention aims at alleviating or even solving at least some of the problems mentioned previously.

The invention is defined by claim 1.

The invention relates to an implant for determining a pressure difference inside a bodily lumen. Such an implant could take the form of a stent or a stent graft that can be implanted inside the patient's vasculature. However, other kinds of implant, such as, for example, heart valves, and stents/stent grafts that can be implanted into other bodily lumens such as the bowels, larynx, urethra,..., are also considered.

The implant comprises an implantable body which is a component that can be installed inside the bodily lumen. This body is arranged so that it can be retained in place inside the bodily lumen, for example by the walls of the implantable body pushing against the walls of the bodily lumen. The implantable body comprises a first conductive element and a second conductive element which are both electrically conductive. In embodiments, they could be made of or comprise a metal. However, other electrically conductive materials (e.g. conductive polymers or carbon fibres) are also envisaged as materials that can be used for the first and second conductive elements. The first and second conductive elements are electrically insulated relative to each other so that electricity cannot travel from the first to the second conductive element or vice versa.

Furthermore, the implant comprises first and second pressure sensors. Such pressure sensors are sensors that can determine the pressure at the place where they are arranged. The first and the second pressure sensors are arranged at different locations relative to the implantable body so that they can be used for determining the pressure difference between those two locations. The types of sensors that are envisaged are Microelectromechanical systems (MEMS) capacitive pressure sensors. They have been widely used for pressure measurement in medical application. MEMS capacitive pressure sensors are passive devices that may not require a power source at all; the excitation signal can be provided by the external reader. This makes them suitable for implanted medical devices. In addition, since they are inherently AC devices, capacitive sensors are suitable for wireless applications.

The first pressure sensor is arranged for sending the pressure that is measured by that first pressure sensor to a remote read-out device by means of the first pressure sensor being coupled to the first conductive element. The first conductive element accordingly serves as an antenna. Accordingly, the first pressure sensor is coupled to the first conductive element, for example using an electrical connection, and can transmit the measured local pressure to a remote read-out device, which can be located outside of the patient's body. This transmission of the measured pressure can occur using wireless communication. However, other ways of sending the pressure value to the remote read-out device are also envisaged.

Also, the second pressure sensor is arranged for sending the pressure measured by that sensor to the remote read-out device by means of the second pressure sensor being coupled to the second conductive element, where the second conductive element serves as an antenna. For the sending of the pressure from the second pressure sensor to the remote read-out device, the same communication modes can be employed that are also used by the first pressure sensor. However, it is not necessary that the first and the second pressure sensor use the same communication protocol.

By the claimed invention, since the first and the second pressure sensors each have their own dedicated antenna in the form of the respective first and second conductive elements, each of the first and second pressure sensors can communicate with the remote read-out device using their dedicated antenna. This means that the communication from the first pressure sensor and the second pressure sensor does not interfere with each other. They will thus suffer comparatively little interference. Furthermore, it is not necessary to precisely synchronise when the first and the second pressure sensors transmit their respective data which would otherwise be necessary if they had a shared antenna (since there could then be issues if they both attempted to transmit their measured pressure at exactly the same time using the same antenna). On the other hand, even if the two sensors do not transmit their data at precisely the same time, it can be advantageous if they both transmit their respective data so close in time (typically within a maximum delay of 3-5 s) to allow for a meaningful pressure difference measurement. Since they do not share the same antenna, it is now also possible for them to transmit at the same time. Accordingly, this feature allows for a simplification of the implant and for a less complicated signal transmission.

Additionally, by the pressure sensors being integrated into the implantable body, and by the read-out device operating remotely and data being transferred wirelessly, it is not necessary to perform a surgery for obtaining the pressure difference. This makes measuring the pressure difference easier since no surgery is required.

In contrary to the conventional TIPS, this invention enables pressure gradient measurements in real time. The measurements can start right from the TIPS procedure to post-procedural monitoring. Hence, this diagnostic system facilitates optimal shunt diameter selection during TIPS procedure. Due to constant monitoring, the pressure gradient trends can be determined during patients' daily life, the data collected could be also correlated with further vital parameters. The success of therapy can be monitored regularly and in the long term. In case that a portal hypertension complication occurs, the prediction is almost instant and allows treatment at a much earlier stage. Ultimately, the diagnostic system can prevent relapse.

In general, this invention is beneficial to both patients and clinics because the diagnostic system is easy to handle, precise and cost-effective. Radiation and contrast exposure can be reduced in the monitoring and thus fewer hospital visits are required. The invention thus has the potential of improving a patient's health and also their quality of life.

In the invention, the implantable body comprises a (preferably tubular) stent that is arranged for being implanted inside a patient's blood vessels. The implant is thus capable of measuring a pressure gradient that occurs inside a patient's blood vessels, which is of high diagnostic relevance.

In the invention, the stent is a wire stent comprising a section made of a helically wound wire. Such stents are highly flexible and can thus be implanted in tortuous vessels.

In the invention, the first conductive element and/or the second conductive element are part of the (same) helically wound wire that forms part of the wire stent. That is, the wire stent has one section that doubles up as being a component that defines - at least in parts - the shape of the stent and that also serves for transmitting data. This makes the implant more compact and simplifies its structure. Furthermore, since the antennas of the first and/or second pressure sensors take the shape of a helix, one obtains an antenna that is, in the context of radio communications, known as a "rubber ducky antenna". Such antennas have a good radiation performance combined with a comparatively short length and are hence useful for devices that should, in a number of embodiments, be miniaturised whilst also being able to communicate with them comparatively easily.

In the invention, the first and the second conductive elements are connected to each other by means of an insulating connector sheath. This improves the structural integrity of the implant whilst also ensuring that the first and the second conductive elements do not come into contact with each other. According to the invention, this allows for both the first and the second conductive elements being part of the same helix, which leads to favourable transmission properties.

In embodiments, the stent further comprises a section made of a woven wire. Such a section makes the stent more flexible.

In embodiments, the first and/or second conductive elements are provided as separate wire sections that are electrically insulated from each other and, in embodiments, from the remainder of the implant. This allows for a greater amount of flexibility in both the design of the stent and the first and/or second conductive elements.

In embodiments, the stent is self-expanding. Such stents, which can comprise a shape memory metal such as nitinol, are comparatively easy to deploy.

In embodiments, the first and second pressure sensors are arranged at opposite longitudinal ends of the stent, where the longitudinal axis of the stent is along the axis of symmetry. This allows for measuring the pressure difference across the largest dimension of the stent and hence leads to more meaningful data.

In embodiments, the stent is at least partially covered with a covering material to form a stent graft. In that scenario, the stent itself plays the role of the base stent of the stent graft.

In further embodiments, the stent graft is a TIPS stent graft. This allows for measuring the portal pressure gradient, which is of high clinical significance for people who have been treated with a TIPS stent graft. Such a TIPS stent graft can be understood to be a stent graft that is only partially covered, where the uncovered part of the base stent is intended for being arranged inside the patient's portal vein when the TIPS stent graft is implanted. Such TIPS stent grafts are sufficiently long to reach from a patient's portal vein to the hepatic vein. In addition, the system can also be applied to PAD, Coronary, AV Access, Venous and AAA and other stents and stent grafts that would benefit from pressure monitoring.

### Brief Description of the Drawings

Figure 1 shows a stent graft according to a first embodiment of the invention.
Figure 2a) shows the stent graft according to a second embodiment of the invention. Figures 2b) and c) show the stent graft according to a third embodiment of the invention.
Figure 3 shows the stent graft according to the first embodiment of the invention, in sub-figures a)-d).

### Detailed Description of the Drawings

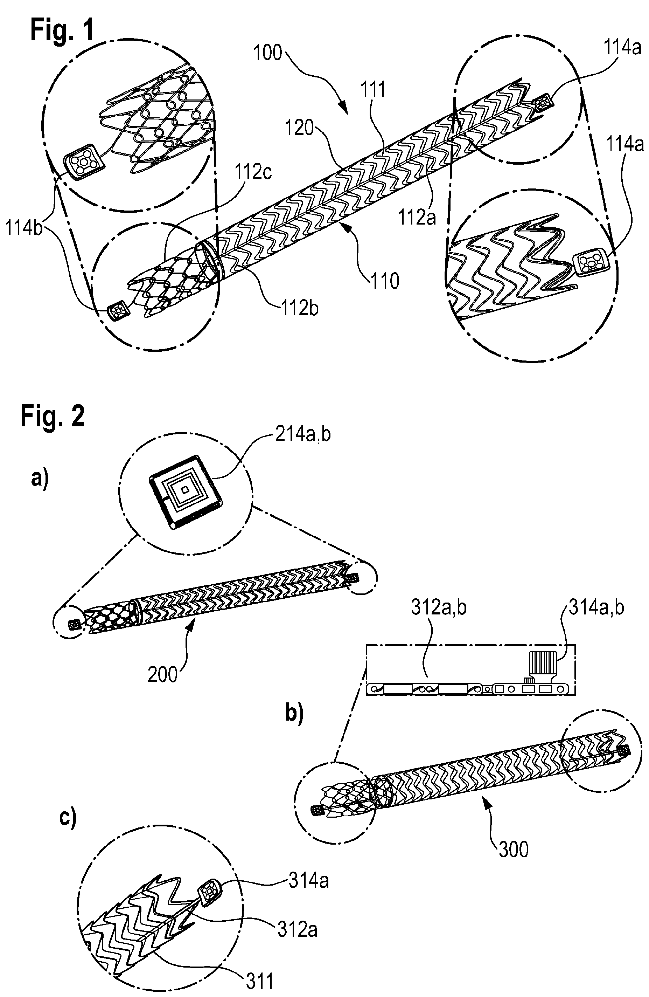

Figure 1 shows a TIPS stent graft 100 according to a first embodiment of the invention. A stent graft body 110 comprises two sections, namely an uncovered stent section 112c and a covered stent graft section 120 where a base stent 111 is covered by a covering material 119 that could, in embodiments, be made of ePTFE or FEP. In the uncovered stent section 112c, the base stent 111 is not covered by the covering material 119.

The base stent 111, which has the shape of a single-lumen tube, is a wire stent. In the stent graft section 120, the wire of the base stent 111 has the form of a single thread of helically wound wire, where the wire is, in addition to being generally helically wound about the longitudinal axis of the stent graft, also zig-zagging along the longitudinal direction. Put differently, the stent graft portion 120 has the wire in the form of a wavy wire that is arranged in a helical shape.

The uncovered stent portion 112c has the wire of the base stent 111 arranged in a woven form so that the wire forms loops that are entangled with each other. Accordingly, the uncovered stent portion 112c, which is intended for being implanted in a patient's portal vein, is more flexible as compared with the stent graft portion 120.

At the respective longitudinal ends of the stent graft 110, first and second pressure sensors 114a, 114b are arranged.

Those pressure sensors 114a, 114b measure the pressure at those respective longitudinal ends.

The first pressure sensor 114a is electrically connected to a first conductive element 112a which is a part of the helically wound wire forming the base stent 111 of the stent graft section 120. This first conductive element 112a serves as an antenna for the first pressure sensor 114a.

Likewise, the second pressure sensor 114b is connected to a second conductive element 112b that is part of the base stent 111 inside the stent graft section 120. The second conductive element 112b is electrically insulated from the first conductive element 112a and is also insulated from the uncovered stent section 112c by means of insulating sheath 116, 118, which are arranged at the junctions of the first/second conductive elements 112a, 112b and of the wire of the uncovered stent section 112c. Both the first and the second conductive element 112a, 112b are covered on the luminal and abluminal side with the covering material 119. The free ends of the wire of the base stent 111 are welded to the body of the base stent itself. The insulating sheaths 116, 118 can, in embodiments, be made of polytetrafluoroethylene (PTFE), which is, in embodiments, press-fit to the connected wires, potentially with a subsequent sintering step. Through PTFE sintering and compression during the sintering process, the constant radial contraction force generated and the friction between PTFE and the wires can allow the PTFE to hold the junctions to the wires.

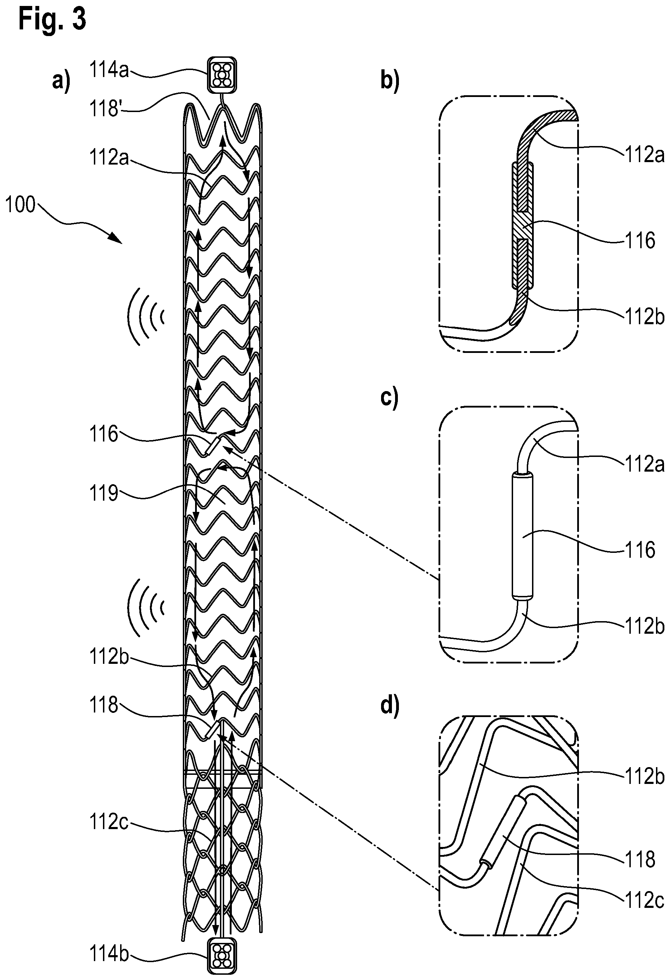

The stent graft 100 according to the first embodiment of the present invention can be seen in more detail in Figures 3a)-d). In those figures, the insulating sheath 116 that is provided between the first and the second conductive elements 112a, 112b can be seen in a longitudinal section (Figure 3b)) and in an outside view (Figure 3c)). Additionally, the insulating sheath 118 with which the second conductive element 112b and the uncovered stent section 112c are connected can also be seen. This second sheath 118 is structurally identical to the insulating sheath 116. The covering material 119 covers both the inside and the outside of the base stent 111 and thus prevents blood from interfering with the transmission of signals from the first and second pressure sensors 114a, 114b. Pressure sensors 114a, 114b use MEMS technology. In order to minimize the disturbance of normal blood flow and allow proper crimping and loading of the stent, first and second pressure sensors 114a, 114b with a very small footprint (1.5 mm²) and a thickness of 0.2mm are used. For sensor integration with the base stent 111, laser micro-welding is used.

As indicated in Figure 3a), the pressure that is sensed by the first pressure sensor 114a will cause an electromagnetic field to be emitted by the antenna formed by the first conductive element 112a, as indicated by the circular arrows. The first pressure sensors 114a will thus communicate with a remote read-out device (not shown) so as to send the sensed pressure value to that device.

Likewise, in the part of the base stent 111 that is formed by the second conductive element 112b, another electromagnetic field as indicated by arrows is created, which is also used for sending information to the remote read-out device. As can be seen from Figure 3a), since the first and the second conductive elements 112a, 112b are electrically insulated by means of the insulating connector sheath 116, there is little, if any, interference between the two antennas, which improves signal transmission.

The overall configuration of a stent graft according to a second embodiment of the invention can be seen in Figure 2a). Again, it can be seen that the first and second pressure sensors 214a, 214b are provided at the respective longitudinal ends. However, in contrast with the first embodiment, the antennas of the first and second pressure sensors 114a, 114b are integrated within those sensors, so that the base stent does not also function as an antenna for them. Apart from this, the structure is identical to that of the first embodiment, with the exception of the fact that the base stent is made of a single piece of wire, rather than three such pieces that are connected to each other via insulating connector sheaths.

A third embodiment of the present invention can be seen in Figures 2b) and c). In the TIPS stent graft 300 according to the third embodiment, similar components are used as in the first embodiment, so that only the differences are discussed.

Whilst in the first embodiment, parts of the base stent of the TIPS stent graft 100 also function as an antenna for the first and second pressure sensors 114a, 114b, in the third embodiment, the first and the second pressure sensors 314a, 314b are each connected to their respective dedicated first and second conductive elements 112a that are electrically isolated from the base stent 111 and that thus do not conduct electricity to the base stent. Accordingly, the function of the antenna is, in the third embodiment, performed solely by the dedicated first and second conductive elements 312a, 312b. Since it is thus not necessary to take into consideration the signal transmission performance of the base stent when designing the base stent 311, and since one also does not have to take into consideration the effect of the antenna shape on the mechanical stability of the base stent 300 when designing the first and second conductive elements 312a and 312b for the stent graft 300, a higher degree of flexibility in the stent graft design can be achieved.

In the embodiments described herein, the base stent was made of nitinol, which has a good electrical connectivity and which is well known as a self-expanding shape memory alloy that is used in medicine. Accordingly, the base stent has favourable mechanical properties as well as to favourable signal transmission properties. As the covering material 119, ePTFE was used, which is a good insulator and hence prevents the blood from interfering with the signal transmission. Given that the blood is a good electrical conductor, it would, if it were to contact the antenna, have a negative effect on signal transmission. In the third embodiment, the first and second conductors 312a, 212b have the form of loop antennas which are embedded inside the covering material 119. They are thus also insulated from blood but also from getting in contact with a patient's blood vessels.

In comparison between the first and the second/third embodiments, the first embodiment has a higher inductance, due to the large cross-sectional area of the antenna. In contrast with this, the second and third embodiments have a somewhat lower inductance. The first embodiment has an inductance variance that is dependent on the stent geometry whilst, for the second and third embodiment, the inductance variance is relatively constant and only depends on the design of the antenna.

The first embodiment is advantageous in the sense that electromagnetic signal will only be scattered by the bodily tissue whereas in the second and third embodiment, it will be interfering with both the body tissues as well as the stent. In the first embodiment, the joint between the sensor and the antenna could be made using a conductive epoxy resin or a laser micro welding whilst, in the case of the second and third embodiment, the joint would be fabricated together with the sensor, which would lead to a better joint quality.

In use, the TIPS stent grafts 100, 200, 300 of the first to third embodiments would be advanced through a patient's vasculature using a suitable delivery platform (generally a catheter) until the uncovered stent section 112c extends into the patient's portal vein whilst the covered stent graft section 120 is arranged inside an artificially created TIPS shunt so as to line that shunt.

If it is desired to read out the pressure difference across the TIPS shunt, the respective first and second pressure sensors are queried using a signal emitted from an external emitter which can, depending on whether the first and second pressure sensors have their own inbuilt power supply, also be used for driving them. The first and second pressure sensors will respond by sending their locally measured pressure values to an external receiver which will then use the received values to thus obtain a pressure difference. This pressure difference can then be used by a medical practitioner to determine whether the TIPS shunt is working well and if further interventions are needed.

## Claims

1. Implant (100, 200, 300) for determining a pressure difference inside a bodily lumen, comprising:
- an implantable body (110), the implantable body comprising a first conductive element (112a, 212a) and a second conductive element (112b, 212b), the first and second conductive elements (112a, 112b, 212a, 212b) being electrically insulated relative to each other,
- first and second pressure sensors (114a, 114b), the first and second pressure sensors (114a, 114b) being arranged for measuring a pressure occurring inside the body, the first and second pressure sensors (114a, 114b) being arranged at different locations,
wherein the first pressure sensor (114a) is arranged for sending the pressure to a remote read-out device by means of being coupled to the first conductive element (112a, 212a) with the first conductive element (112a, 212a) serving as an antenna, and wherein the second pressure sensor (114b) is arranged for sending the pressure to a remote read-out device by means of being coupled to the second conductive element (112b, 212b), with the second conductive element (112b, 212b) serving as an antenna, wherein the implantable body (110) comprises a stent,
**characterized in that**
the stent (110) is a wire stent comprising a section made of a helically wound wire, wherein the first conductive element (112a) and the second conductive element (112b) are part of the helically wound wire, wherein the first conductive element (112a) and the second conductive element (112b) are connected to each other by means of an insulating connector sheath (116) so that the first and second conductive elements (112a, 112b) are part of the same helix.

2. Implant according to claim 1, wherein the first conductive element (112a) and/or the second conductive element (112b) are made of a single piece of wire.

3. Implant according to one of claims 1 to 2, wherein the stent further comprises a section made of a woven wire (112c).

4. Implant according to one of claims 1 to 3, the stent being self-expanding.

5. Implant according to one of claims 1 to 4, wherein the first and the second pressure sensors (114a, 114b) are arranged at opposite longitudinal ends of the stent.

6. Implant according to one of claims 1 to 5, wherein the stent is at least partially covered with a covering material (119) to form a stent graft.

7. Implant according to claim 6, wherein the stent graft is a TIPS stent graft.

## Patentansprüche

1. Implantat (100, 200, 300) zur Bestimmung einer Druckdifferenz innerhalb eines Körperlumens, umfassend:
- einen implantierbaren Körper (110), wobei der implantierbare Körper ein erstes leitfähiges Element (112a, 212a) und ein zweites leitfähiges Element (112b, 212b) umfasst, wobei das erste und zweite leitfähige Element (112a, 112b, 212a, 212b) elektrisch voneinander isoliert sind,
- erste und zweite Drucksensoren (114a, 114b), wobei die ersten und zweiten Drucksensoren (114a, 114b) angeordnet sind, um einen im Inneren des Körpers auftretenden Druck zu messen, wobei die ersten und zweiten Drucksensoren (114a, 114b) an unterschiedlichen Stellen angeordnet sind,
wobei der erste Drucksensor (114a) dazu angeordnet ist, den Druck an eine entfernte Auslesevorrichtung zu senden, indem er mit dem ersten leitfähigen Element (112a, 212a) gekoppelt ist, wobei das erste leitfähige Element (112a, 212a) als Antenne dient, und wobei der zweite Drucksensor (114b) dazu angeordnet ist, den Druck an eine entfernte Auslesevorrichtung zu senden, indem er mit dem zweiten leitfähigen Element (112b, 212b) gekoppelt ist, wobei das zweite leitfähige Element (112b, 212b) als Antenne dient, wobei der implantierbare Körper (110) einen Stent umfasst,
**dadurch gekennzeichnet, dass**
der Stent (110) ein Drahtstent ist, der einen Abschnitt aus einem spiralförmig gewickelten Draht umfasst, wobei das erste leitfähige Element (112a) und das zweite leitfähige Element (112b) Teil des spiralförmig gewickelten Drahtes sind, wobei das erste leitfähige Element (112a) und das zweite leitfähige Element (112b) mittels einer isolierenden Verbindungshülse (116) miteinander verbunden sind, so dass das erste und zweite leitfähige Element (112a, 112b) Teil derselben Spirale sind.

2. Implantat nach Anspruch 1, wobei das erste leitfähige Element (112a) und/oder das zweite leitfähige Element (112b) aus einem einzigen Stück Draht hergestellt sind.

3. Implantat nach einem der Ansprüche 1 bis 2, wobei der Stent weiter einen Abschnitt umfasst, der aus einem Drahtgewebe (112c) hergestellt ist.

4. Implantat nach einem der Ansprüche 1 bis 3, wobei der Stent selbstexpandierend ist.

5. Implantat nach einem der Ansprüche 1 bis 4, wobei der erste und der zweite Drucksensor (114a, 114b) an gegenüberliegenden Längsenden des Stents angeordnet sind.

6. Implantat nach einem der Ansprüche 1 bis 5, wobei der Stent zumindest teilweise mit einem Abdeckmaterial (119) bedeckt ist, um einen Stentgraft zu bilden.

7. Implantat nach Anspruch 6, wobei der Stentgraft ein TIPS-Stentgraft ist.

## Revendications

1. Implant (100, 200, 300) pour déterminer une différence de pression à l'intérieur d'un conduit corporel, comprenant :
- un corps implantable (110), le corps implantable comprenant un premier élément conducteur (112a, 212a) et un second élément conducteur (112b, 212b), les premier et second éléments conducteurs (112a, 112b, 212a, 212b) étant isolés électriquement l'un par rapport à l'autre,
- des premier et second capteurs de pression (114a, 114b), les premier et second capteurs de pression (114a, 114b) étant conçus pour mesurer une pression se produisant à l'intérieur du corps, les premier et second capteurs de pression (114a, 114b) étant placés à différents emplacements,
dans lequel le premier capteur de pression (114a) est conçu pour envoyer la pression à un dispositif de lecture distant au moyen d'un couplage au premier élément conducteur (112a, 212a), le premier élément conducteur (112a, 212a) servant d'antenne, et dans lequel le second capteur de pression (114b) est conçu pour envoyer la pression à un dispositif de lecture distant au moyen d'un couplage au second élément conducteur (112b, 212b), le second élément conducteur (112b, 212b) servant d'antenne, le corps implantable (110) comprenant un stent,
**caractérisé en ce que**
le stent (110) est un stent en fil métallique comprenant une portion constituée d'un fil enroulé en hélice, dans lequel le premier élément conducteur (112a) et le second élément conducteur (112b) font partie du fil enroulé en hélice, dans lequel le premier élément conducteur (112a) et le second élément conducteur (112b) sont reliés l'un à l'autre au moyen d'une gaine de connecteur isolante (116) de telle sorte que les premier et second éléments conducteurs (112a, 112b) font partie de la même hélice.

2. Implant selon la revendication 1, dans lequel le premier élément conducteur (112a) et/ou le second élément conducteur (112b) sont constitués d'une seule pièce de fil.

3. Implant selon l'une des revendications 1 à 2, dans lequel le stent comprend en outre une section en fil tissé (112c).

4. Implant selon l'une des revendications 1 à 3, le stent étant auto-expansible.

5. Implant selon l'une des revendications 1 à 4, dans lequel les premier et second capteurs de pression (114a, 114b) sont disposés à des extrémités longitudinales opposées du stent.

6. Implant selon l'une des revendications 1 à 5, dans lequel le stent est au moins partiellement recouvert d'un matériau de recouvrement (119) pour former un greffon sous forme de stent.

7. Implant selon la revendication 6, dans lequel le greffon sous forme de stent est un greffon sous forme de stent TIPS.
